# EUROPÄISCHE PATENTSCHRIFT

(11) **EP 2 170 855 B1**
(45) Veröffentlichungstag und Bekanntmachung des Hinweises auf die Patenterteilung: **08.08.2012**
(21) Anmeldenummer: 08774262.3
(22) Anmeldetag: 24.06.2008
(51) Int. Cl.: C07D 301/30, C07D 303/16, C09D 5/02, C09D 7/02

(54) **C10 ALKANSÄUREGLYCIDESTER UND IHRE VERWENDUNG**
C10 ALKANOIC ACID GLYCIDYL ESTERS AND USE THEREOF
ESTER GLYCIDIQUE D'UN ACIDE ALCANOÏQUE EN C10 ET SON UTILISATION

(30) Priorität: 28.06.2007 EP 07111275
(43) Veröffentlichungstag der Anmeldung: 07.04.2010
(73) Patentinhaber: BASF SE, 67056 Ludwigshafen (DE)
(72) Erfinder: GUMLICH, Kai, 68159 Mannheim (DE); MERTEN, Roland, 69469 Weinheim (DE); HENNINGSEN, Michael, 67227 Frankenthal (DE); SCHÄFER, Martin, 67269 Grünstadt (DE); TELES, Joaquim, Henrique, 67166 Otterstadt (DE); MOHR, Bernhard, 67150 Niederkirchen (DE)
(86) Internationale Anmeldenummer: PCT/EP2008/058036
(87) Internationale Veröffentlichungsnummer: WO 2009/000839

(56) Entgegenhaltungen:
- WO-A-00/44836
- BUKOWSKA ET AL: "Synthesis of glycidyl esters" J. CHEM. TECHNOL. BIOTECHNOL., Bd. 74, 1999, Seiten 1145-1148, XP002494101 in der Anmeldung erwähnt

## Beschreibung

Die Erfindung betrifft 2-Propylheptansäureglycidester, 4-Methyl-2-propylhexansäureglycidester oder deren Gemisch (zusammenfassend kurz als Glycidester bezeichnet).

Glycidester haben aufgrund ihrer physikalischen und chemischen Eigenschaften vielfältige Verwendungsmöglichkeiten. Sie werden z.B. als Reaktivverdünner für Polyacrylate, Polyester oder Epoxidsysteme verwendet, sind als Dispergierhilfsmittel, z.B. für Pigmente geeignet oder werden als Zwischenprodukte in Synthesen, insbesondere für die Modifizierung von Oligomeren und Polymeren eingesetzt. Bekannt sind insbesondere verzweigte C 10 Alkansäureclycidester, z.B. Glycidester der Versaticsäure, wie sie unter dem Handelsnamen Cadura® E 10 von Shell erhältlich sind oder Glycidester der Neodecansäure wie sie unter der Bezeichnung Glydexx® von Exxon erhältlich sind.

Agnieszka Bukowska et al., J. Chem.Technol Biotechnol 74 ; 1145-1148 (1999) beschreiben die Herstellung von Glycidylestern durch Umsetzung der entsprechenden Carbonsäuäuren mit Epichlorhydrin in Gegenwart von Chrom (II)salzen und anschließenden Ringschluss zum Glycidylester unter verwendung von Acetonitril und Kaliumcarbonat. Das Produkt wird durch Destillation gereinigt.

US 6433217 offenbart die Herstellung von am alpha C -Atom verzeigten Glycidylestern, insbesondere Estern der Versaticsäure, durch Umsetzung der Carbonsäure mit Epichlorhydrin in Gegenwart eines Katalysators, anschließende Zugabe eines Alkalimetallhydroxids und Aufarbeitung durch Destillation.

Verwendungen der Glycidester als Reaktivverdünner oder Dispergiergilfsmittel z.B. für Pigmente oder zur Modifizierung von Polymeren durch polymeranaloge Umsetzung sind im Markt bekannt. EP 1042 402 beschreibt z.B. eine Verwendung in Beschichtungszusammensetzungen.

Es besteht ein Bedarf an alternativen Glycidestern mit möglichst guten anwendungstechnischen Eigenschaften und möglichst einfacher und kostengünstiger Herstellung.

Demgemäß wurden die oben definierten Glycidester und ihre Verwendung gefunden. Gefunden wurde auch ein Verfahren zu ihrer Herstellung.

Zu den Glycidestern und ihrer Herstellung

Bei den erfindungsgemäßen Glycidestern handelt es sich um 2-Propylheptansäureglycidester der Formel 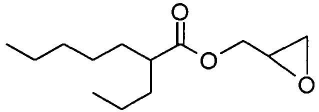 oder 4-Methyl-2-propylhexansäureglycidester der Formel 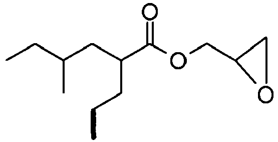 oder ein Gemisch der beiden.

Die erfindungsgemäßen Glycidester sind bei 1 bar, 21 °C flüssig.

2-Propylheptansäureglycidester, 4-Methyl-2-propylhexansäureglycidester oder ein Gemisch der beiden wird leicht durch Dimerisierung von Pentenen und anschließende Oxidation gewonnen, wie es in DE-A 102 39 134 beschrieben ist. Bei der Dimerisierung des Pentens entsteht im allgemeinen ein Gemisch aus 2-Propylhepten und 4-Methyl-2-propylhexen. Das Gemisch kann aufgearbeitet werden und die reinen Verbindungen können dann zu den Carbonsäuren oxidiert und weiter verwendet werden.

Im allgemeinen besteht hierzu jedoch keine Notwendigkeit, da sich beide Carbonsäurenund beide daraus abgeleiteten Glycidester für die weiteren Verwendungen im Rahmen dieser Erfindung eignen. Vorzugsweise wird daher das Gemisch ohne Auftrennung der Alkene zu dem entsprechenden Carbonsäuregemisch oxidiert. Bei der Dimerisierung und anschließenden Oxidation können weitere Nebenprodukte entstehen, z.B. Ameisensäureester, wie Ameisensäure 1-propyl-hexylester oder Ameisensäure 3-methyl-1-propyl-pentylester, Gemische von Nonanol bzw. 6-Methyl-octan-4-ol und Ameisensäure, eine Abtrennung dieser Nebenprodukte vor der Herstellung und weiteren Verwendung der Glycidester ist nicht notwendig.

Die Menge dieser Nebenprodukte ist aber vorzugsweise kleiner als 20 Gew.-Teile, insbesondere kleiner als 10 Gew.-Teile, besonders bevorzugt kleiner 5 Gew.-Teil auf 100 Gew.-Teile 2-Propylheptansäure, 4-Methyl-2-propylhexansäure oder deren Gewichtssumme.

Besonders bevorzugt handelt es sich bei dem erfindungsgemäßen Glycidester um ein Gemisch aus 2-Propylheptansäure- und 4-Methyl-2-propylhexansäure-glcidester.

Besonders bevorzugt ist der Anteil der beiden Verbindungen dabei
1 bis 99 Gew.-% 2-Propylheptansäureglycidester und
1 bis 99 Gew.-% 4-Methyl-2-propylhexansäureglycidester
wobei die Prozentangabe auf die Summe der beiden bezogen ist..
Besonders bevorzugt besteht das Gemisch aus
10 bis 90, insbesondere 20 bis 80 Gew.-% 2-Propylheptansäureglycidester und
10 bis 90, insbesondere 20 bis 80 Gew.-% 4-Methyl-2-propylhexansäureglycidester.

Als Verfahren zur Herstellung der erfindungsgemäßen Glycidester kommen z.B. folgende Verfahren in Betracht:
Das in DE-A 2107084 beschriebene verfahren der Umesterung von Carbonsäuremethylester mit Glycidylacetat oder -propionat mit Natriummethylat oder Ammoniumhydroxide als Katalysatoren; das enstehende Methylacetat wird abdestilliert.

Eine Umsetzung von Carbonsäure mit einer siedenden Mischung aus Epichlorhydrin (2,5 facher molarer Überschuss) und Natriumcarbonat (0,5 mol bzgl. der Säure). Gemäß DE-A 2446944. Im Wasserabscheider trennen sich Reaktionswasser und Epichlorhydrin. Epichlorhydrin wird zurückgeführt. Wenn das gesamte Wasser entfernt wurde, erfolgt die Zugabe von Triethylbenzylammoniumchlorid, danach wird bei 110-115°C gerührt und nach Filtration (NaCl) destillativ aufgearbeitet.

Ein weiteres Verfahren ist die Veresterung von Carbonsäuren mit Glycidol mit Carbodiimiden (z.B. DCC) als Acylierungsreagenzien und Pyridinen (z.B. DMAP) als Katalysatoren gemäß EP-A 0697018. Harnstoff wird abfiltriert, der Katalysator wird mit einem sauren Ionentauscher entfernt. Mit 50%iger wässriger Essigsäure wird restliches Carbodiimid zerstört. Lösungsmittel wird im Vakuum entfernt. Rohprodukt wird in organischem Lösungsmittel aufgenommen und durch Kühlen restlicher Harnstoff auskristallisiert. Lösungsmittel wird unter vermindertem Druck abdestilliert. Produkt verbleibt als Rückstand.

Gemäß US 5036154 erfolgt die Umsetzung durch mit Natriumwolframat katalysierte Epoxidierung von Allylestern mit 70%igem H₂O₂. Es handelt sich um eine 2-phasige Reaktion (Wasser/Toluol) mit Trioctylammoniumchlorid als Phasentransferkatalysator (PTC).

EP 1115714 beschreibt die Umsetzung von Neodecansäre mit einem ca. 4fachen molaren Überschuss Epichlorhydrin in Gegenwart von Isopropanol (wassermischbarer Allkohol), Wasser und NaOH.

Neodecansäure, Epichlorhydrin und die Lösungsmittel werden vorgelegt, dann wird bei 56°C 50%ige NaOH zudosiert. Temperatur wird auf 84°C erhöht, dann abgekühlt auf 50°C. Nach Abtrennen der unteren Phase wird bei 50°C 24%ige NaOH zudosiert. Nach 40 min erfolgt Phasentrennung. Die organische Phase wird bis zu den Endbedingungen von 100mbar, 110°C eingeengt. Überschüssiges Epichlorhydrin wird durch Wasserdampfdestillation entfernt. Dann Zugabe von 50%iger NaOH bei 55°C. Nach Reaktionszeit zweimalige Extraktion mit Wasser, Organische Phase wird mit Dampf gestrippt und im Vakuum getrocknet (120°C, 40 mbar).

Gemäß WO 00/44836 wird aus 1 mol 2-Ethylhexansäure und 2 mol NaOH das Na-Salz der Säure in Toluol hergestellt. Entstehendes Wasser wird mit Hilfe des Toluols azeotrop abdestilliert. Bei 50°C werden 3 mol Epichlorhydrin langsam zugegeben. Die Mischung wird zum Rückfluss erhitzt. Nach Reaktionsende wird das Epichlorhydrin unter abdestilliert. Das Produkt wird unter vermindertem Druck destilliert.

Ein bevorzugtes Verfahren zur Herstellung der erfindungsgemäßen Glycidester ist durch folgende Verfahrensschritte a) bis c) gekennzeichnet:
a) 2-Propylheptansäure, 4-Methyl-2-propylhexansäure oder deren Gemische wird mit Epichlorhydrin in Gegenwart eines Chromsalzes umgesetzt
b) die erhaltene Hydroxyverbindung wird in Gegenwart eines Alkali- oder Erdalkalihydroxids zum Glycidester umgesetzt und
c) das erhaltene Gemisch wird anschließend gegebenenfalls extraktiv aufgearbeitet.

Im Verfahrensschritt a) wird Epichlorhydrin vorzugsweise in Mengen von 0,9 bis 2 Mol, besonders bevorzugt in Mengen von 1 bis 1,5 Mol, bezogen auf 1 Mol Alkancarbonsäure eingesetzt.

Bei dem Chromsalz kann es sich um ein beliebiges Salz des Chrom(II) oder Chrom(III) handeln, in Betracht kommen insbesondere Carbonsäuresalze (Alkanoate), z.B. Chrom(II)acetat oder Chrom(II)ethylhexanoat oder Chrom(II)hydroxide oder auch gemischte Anionen (Hydroxid und Alkanoat).

Das Chromsalz wird vorzugsweise in Mengen von 0,0001 bis 0,01 Mol, besonders bevorzugt von 0,0005 bis 0,005 Mol, bezogen auf 1 Mol Alkancarbonsäure eingesetzt.

Die Umsetzung in Verfahrensschritt a) wird vorzugsweise bei Normaldruck (1 bar) und bei einer Temperatur von 30 bis 100°C, vorzugsweise 60 bis 90°C durchgeführt. Bei der Umsetzung kann ein Lösemittel mitverwendet werden; im allgemeinen ist aber ein Lösemittel nicht erforderlich.

Die Umsetzung in Verfahrensschritt a) wird dabei vorzugsweise durch Gaschromatographie überwacht; vorzugsweise erfolgt eine vollständige Umsetzung der Alkancarbonsäure zur Hydroxyverbindung.

Verfahrensschritt b) schließt sich vorzugsweise direkt an Verfahrenschritt a) an, ohne dass ein Aufarbeitung nach Abschluss der Umsetzung in Verfahrensschritt a) erfolgt.

Bei dem Alkali- oder Erdalkalihydroxid kann es sich um ein beliebige Hydroxid, vorzugsweise um ein Alkalihydroxid wie NaOH oder KOH handeln.

Das Alkali- oder Erdalkalihydroxid wird vorzugsweise in Mengen von 1 bis 2 Mol, bezogen auf 1 Mol der in Verfahrensschritt a) erhaltene Hydroxyverbindung eingesetzt, die molare Menge entspricht dabei vorzugsweise der eingesetzten Menge der Alkancarbonsäure, da in Verfahrensschritt a) vorzugsweise eine vollständige Umsetzung erfolgt.

Die Umsetzung in Verfahrensschritt b) wird vorzugsweise ebenfalls bei Normaldruck (1 bar) und bei einer Temperatur von 30 bis 100°C, vorzugsweise 30 bis 80°C durchgeführt.

Die Zutropfgeschwindigkeit wird vorzugsweise so gewählt, dass Temperatur im gewählten Bereich bleibt, nach Beendigung der Zugabe des Alkali - oder Erdalkalihydroxids wird vorzugsweise noch einige Zeit, z.B. zwei Stunden, gerührt. Eine Kontrolle der Umsetzung kann vorzugsweise gaschromatographisch erfolgen.

Anschließend erfolgt vorzugsweise eine extraktive Aufarbeitung des nach Verfahrensschritt b) erhaltenen Gemischs. Besonders bevorzugt erfolgt eine extraktive Aufarbeitung mit Wasser. Die erhaltene wässrige Phase und organische Phase werden getrennt und die organische Phase getrocknet, vorzugsweise durch Zugabe eines Trockenmittels wie Natriumsulfat.

Der Glycidester kann bei dem erfindungsgemäßen Verfahren in großer Reinheit und Ausbeute erhalten werden. Mit dem erfindungsgemäßen Verfahren steht zudem eine sehr einfache und kostengünstige Methode zur Synthese der Alkancarbonsäuren zur Verfügung.

### Zur Verwendung

Die erfindungsgemäßen Glycidester eignen sich z.B. als Reaktivverdünner. Reaktivverdünner bewirken bei Zugabe zu einer Zusammensetzung eine Verminderung der Viskosität, bei der späteren Aushärtung der Zusammensetzung werden sie an Komponenten der Zusammensetzung, welche mit den Reaktivverdünnern reagieren können, gebunden, so dass sie Bestandteil des erhaltenen Produkts, z.B. einer Beschichtung oder eines Formkörpers werden.

Bei der Verwendung der Glycidester als Reaktivverdünner sollte die Zusammensetzung Verbindungen enthalten, die mit der Glycidgruppe reaktiv sind, z.B. sollte die Verbindungen Carbonsäuregruppen, Hydroxylgruppen oder primäre oder sekundäre Aminogruppen enthalten. Bei der Härtung der Zusammensetzung, z.B. durch Temperaturerhöhung oder Bestrahlung mit energiereichem Licht, findet dann die entsprechende Anbindung statt.

Die Zusammensetzungen, welche den Glycidester enthalten, sollten vorzugsweise bei 21 °C, 1 bar flüssig sein und härtbar sein. Insbesondere handelt es sich um thermisch härtbare Beschichtungszusammensetzungen.

Die Zusammensetzungen enthalten vorzugsweise mindestens 0,1 Gew.-%, besonders bevorzugt mindestens 0,5 Gew.-%, ganz besonders bevorzugt mindestens 1 Gew.-% oder auch mindestens 3 Gew.-% bzw. in einer besonderen Ausführungsform mindestens 5 Gew.-% des erfindungsgemäßen Glycidesters; die Menge des Glycidesters übersteigt aber im allgemeinen nicht 50 Gew.-% vorzugsweise nicht 30 Gew.-% bzw. nicht 20 Gew.-%. Die Prozentangaben beziehen sich dabei auf alle Bestandteile der Zusammensetzung mit Ausnahme von Wasser und organischen Lösemitteln.

Als Verbindungen, die mit der Glycidgruppe reaktiv sind kommen niedermolekulare Verbindungen, aber auch oligomere oder polymere Verbindungen in Betracht.

Als polymere Verbindungen seien z.B. durch radikalische Polymerisation erhältliche Polymere, wie Polyacrylate oder Polyvinylester, Polyester oder Polyurethane genannt, die einen Gehalt an mit dem Glycidester reaktiven Gruppen, z.B. Carbonsäuregruppen, Hydroxylgruppen oder Aminogruppen aufweisen.

Geeignete Zusammensetzungen enthalten insbesondere übliche Epoxide, worunter im folgenden Verbindungen mit mindestens einer Epoxidgruppe verstanden werden, abgesehen von den erfindungsgemäßen Glycidestern. Der Glycidester enthält ebenfalls Epoxygruppen. Die Epoxide und der Glycidester können dann in gleicher Weise, z.B. durch Zusatz eines Amin- oder eines Anhydridhärters aushärten.

Besonders bevorzugt enthält die härtbare Zusammensetzung Epoxide mit 2 bis 6, ganz besonders bevorzugt mit 2 bis 4 und insbesondere mit 2 Epoxygruppen.

Bei den Epoxygruppen der Epoxide handelt es sich insbesondere um Glycidylethergruppen, wie sie bei der Umsetzung von Alkoholgruppen mit Epichlorhydrin entstehen.

Bei den Epoxiden kann es sich um niedermolekulare Verbindungen, welche im allgemeinen ein mittleres Molgewicht Mn kleiner 1000 g/mol haben oder um höhermolekulare Verbindungen (Polymere) handeln. Es kann sich um aliphatische, auch cycloaliphatische Verbindungen oder um Verbindungen mit aromatischen Gruppen handeln.

Insbesondere handelt es sich bei den Epoxiden um Verbindungen mit zwei aromatischen oder aliphatischen 6-Ringen oder deren Oligomere.

Technisch von Bedeutung sind Epoxide, die durch Umsetzung des Epichlorhydrins mit Verbindungen, welche mindestens zwei reaktive H-Atome haben, insbesondere mit Polyolen, erhältlich sind.

Technisch von besonderer Bedeutung sind Epoxide, die durch Umsetzung des Epichlorhydrins mit Verbindungen, welche mindestens zwei, vorzugsweise zwei Hydroxylgruppen und zwei aromatische oder aliphatische 6-Ringe enthalten, erhältlich sind; als derartige Verbindungen genannt sei insbesondere Bisphenol A und Bisphenol F, sowie hydriertes Bisphenol A und Bisphenol F.

In Betracht kommen auch Umsetzungsprodukte des Epichlorhydrins mit anderen Phenolen, z.B. mit Kresolen oder Phenol-aldehyd-adukten, wie Phenolformaldehydharzen, insbesondere Novolaken.

Es sind natürlich auch Epoxide geeignet, welche ihre Epoxygruppen nicht vom Epichlorhydrin ableiten. In Betracht kommen z.B.Epoxide, welche Epoxgruppen durch Umsetzung mit Glycidyl (meth)acrylat), z.B. radikalische Copolymerisation mit Glycidyl (meth)acrylat, enthalten. Genannt sei in diesem Zusammenhang auch ERL-4221 von Dow (CAS Nummer 2386-87-0):

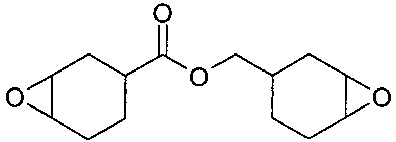

Für die Verwendung der Zusammensetzungen sind insbesondere bei Verarbeitungstemperaturen von 20 bis 100°C, besonders bevorzugt bei 20 bis 40°C, ganz besonders bevorzugt bei 20°C flüssige Epoxide geeignet. Feste Epoxyde können in einem geeigneten Lösemittel oder vorzugsweise auch in dem erfindungsgemäßen Glycidester als Reaktivverdünner gelöst werden.

Die Zusammensetzung enthält im Falle der Epoxidverbindungen vorzugsweise mindestens auch noch einen Härter.

In Betracht kommen Anhydrid-vernetzer wie z.B. Phthalsäureanhydrid, Trimellitsäureanhydrid, Benzophenontetracarbonsäure-dianhydrid, Tetrahydrophthalsäureanhydrid, Hexahydrophthalsäureanhydrid, 4-Methyl-tetrahydrophthalsäureanhydrid, 3-Methyl-tetrahydrophthalsäureanhydrid, 4-Methyl-hexaydrophthalsäureanhydrid oder 3-Methylhexahydrophthalsäureanhydrid.

In Betracht kommen insbesondere auch Amine, bekannte Aminvernetzer sind insbesondere aliphatische Polyamine wie Diethylentriamin Triethylentetra-amin oder Amine auf Basis von Propylenoxid und Ammoniak (Polyetheramine).

Je nach Reaktivität der Vernetzer können diese bereits frühzeitig zur Zusammensetzung gegeben werden (einkomponentiges, bzw. 1-K System) oder erst kurz vor der Verwendung (zweikomponentiges, bzw. 2 K-System).

Bevorzugte Zusammensetzungen bestehen zu mindestens 30 Gew.-%, vorzugsweise zu mindestens 50 Gew.-%, ganz besonders bevorzugt zu mindestens 70 Gew.-% aus Epoxidverbindungen. (abgesehen von ggf. mitverwendeten Lösemitteln).

In einer bevorzugten Ausführungsform enthält die Zusammensetzung inerte Lösemittel allenfalls in untergeordneten Mengen (kleiner 20 Gew.-Teile, insbesondere kleiner 10 bzw. kleiner 5 Gew.-Teile auf 100 Gew.-Teile Epoxidverbindung) und besonders bevorzugt kein Lösemittel (100 % system).

Die Epoxide enthaltenden Zusammensetzungen eignen sich z.B. als Beschichtungs- oder Imprägnierungsmittel, als Klebstoff, als Verbundwerkstoff, zur Herstellung von Formkörpern oder als Gießmassen zur Einbettung, Anbindung oder Verfestigung von Formkörpern. Dies und die nachstehenden Ausführungen dazu gelten sowohl für die 1 K - als auch für 2 K- Systeme..

Als Beschichtungsmittel seien z.B. Lacke genannt. Insbesondere können mit den erfindungsgemäßen Zusammensetzungen (1 K oder 2 K) kratzfeste Schutzlacke auf beliebigen Substraten, z.B. aus Metall, Kunststoff oder Holzwerkstoffen erhalten werden. Die Zusammensetzungen eignen sich auch als Isolierbeschichtungen in elektronischen Anwendungen, z.B. als Isolierbeschichtung für Drähte und Kabel. Genannt sei auch die Verwendung zur Herstellung von Photoresisten. Sie eignen sich insbesondere auch als Reparaturlack, z.B. auch bei der Ausbesserung von Rohren ohne Demontage der Rohre (cure in place pipe (CIPP) rehabilitation). Sie eignen sich auch zur Versiegelung von Fußböden.

Als Klebstoffe seien 1 K oder 2 K- Strukturklebstoffe erwähnt. Strukturklebstoffe dienen zur dauerhaften Verbindung von Formteilen miteinander. Die Formteile können aus beliebigem Material sein; in Betracht kommen Materialien aus Kunststoff, Metall, Holz, Leder, Keramik etc. Es kann sich dabei auch um Schmelzklebstoffe (hot melt adhesives) handeln, die erst bei höherer Temperatur fließfähig und verarbeitbar sind. Es kann sich auch um Fußbodenklebstoffe handeln. Die Zusammensetzungen eignen sich auch als Klebstoffe für die Herstellung von Leiterplatten (electronic curcuits), insbesondere auch nach der SMT Methode (surface mounted technology).

In Verbundwerkstoffen (Composites) sind unterschiedliche Materialien, z.B. Kunststoffe und Verstärkungsmaterialien (Fasern, Carbonfasern) miteinander verbunden.

Die Zusammensetzungen eignen sich z.B. zur Herstellung von vorimprägnierter Fasern, z.B. Prepregs und ihrer weiteren Verarbeitung zu Verbundwerkstoffen.

Als Herstellverfahren für Verbundwerkstoffe seien die Härtung von vorimprägnierten Fasern oder Fasergeweben (z.B. Prepregs) nach Lagerung oder aber die Extrusion, Strangziehen (pultrusion), Wickeln (winding) und resin transfer molding (RTM), resin infusion technologies (RI) genannt.

Insbesondere können die Fasern mit der erfindungsgemäßen Zusammensetzung getränkt werden und danach bei einer höheren Temperatur gehärtet werden. Während der Tränkung und gegebenenfalls einer anschließenden Lagerung setzt noch keine oder nur eine geringfügige Härtung ein.

Als Gießmassen zur Einbettung, Anbindung oder Verfestigung von Formkörpern werden die Zusammensetzungen z.B. in elektronischen Anwendungen eingesetzt. Sie eignen sich als Flip-chip underfill oder als Elektrogießharze für potting, casting und (glob-top-) encapsulation.

Die mit den Zusammensetzungen hergestellten Beschichtungen oder Formkörper oder sonstigen Produkte zeichnen sich durch sehr gute anwendungstechnische Eigenschaften aus, insbesondere durch eine hohe Festigkeit, Kratzfestigkeit, eine gute Chemikalienbeständigkeit, eine gute Härte und Elastizität.

Die erfindungsgemäßen Glycidester eignen sich auch als Dispergier- oder Emulgierhilfsmittel.

In Betracht kommt insbesondere eine Verwendung als Dispergierhilfsmittel für Pigmente. Pigmentzusammensetzungen, wie Pigmentpasten oder oder Pigmentdispersionen, enthalten vorzugsweise 0,1 bis 30 Gew.-Teile, besonders bevorzugt 0,05 bis 20 Gew.-Teile des Glycidesters, bezogen auf 100 Gew.-Teile Pigment.

Weiterhin eignet sich der Glycidester zur chemischen Modifizierung von niedermolekularen aber auch von polymeren Verbindungen. Wesentliche Voraussetzung ist, dass die Verbindung eine funktionelle Gruppe enthält, die mit der Glycidgruppe des Glycidesters reaktiv ist. In Betracht kommen wie bereits oben aufgeführt Hydroxylgruppen, primäre oder sekundäre Aminogruppen sowie Carboxylgruppen. Die so modifizierten niedermolekularen oder polymeren Verbindungen eignen sich z. B. als Bindemittel, insbesondere als Bindemittel in Lacksystemen. Es kann sich um wasser- und lösemittelfreie Bindemittel, z.B. auch um strahlungshärtbare Bindemittel handeln. Bevorzugte Bindemittel sind Polyacryltharze, welche zu mehr als 40 Gew.-%, insbesondere zu mehr als 60 Gew.-% aus C₁-C₂₀-Alkyl(meth)acrylaten bestehen.

Insbesondere eignet sich Glycidester zur Modifizierung von durch radikalische Polymerisation erhältlichen Polymeren, z.B. Polyacrylate, von Polykondensaten, z.B. Polyester, oder Polyaddukten, z.B. Polyurethane.

Geeignete durch radikalische Polymerisation von ungesättigten Verbindungen (Monomere) erhältliche Polymere bestehen vorzugsweise zu mindestens 40 Gew.-%, bevorzugt zu mindestens 60 Gew.-%, besonders bevorzugt zu mindestens 80 Gew.-% aus sogenannten Hauptmonomeren.

Die Hauptmonomeren sind ausgewählt aus C₁-C₂₀-Alkyl(meth)acrylaten, Vinylestern von bis zu 20 C-Atome enthaltenden Carbonsäuren, Vinylaromaten mit bis zu 20 C-Atomen, ethylenisch ungesättigten Nitrilen, Vinylhalogeniden, Vinylethern von 1 bis 10 C-Atome enthaltenden Alkoholen, aliphatischen Kohlenwasserstoffen mit 2 bis 8 C-Atomen und ein oder zwei Doppelbindungen oder Mischungen dieser Monomeren.

Zu nennen sind z.B. (Meth)acrylsäurealkylester mit einem C₁-C₁₀-Alkylrest, wie Methylmethacrylat, Methylacrylat, n-Butylacrylat, Ethylacrylat und 2-Ethylhexylacrylat.

Insbesondere sind auch Mischungen der (Meth)acrylsäurealkylester geeignet.

Vinylester von Carbonsäuren mit 1 bis 20 C-Atomen sind z.B. Vinyllaurat, -stearat, Vinylpropionat, Versaticsäurevinylester und Vinylacetat.

Als vinylaromatische Verbindungen kommen Vinyltoluol, α- und p-Methylstyrol, α-Butylstyrol, 4-n-Butylstyrol, 4-n-Decylstyrol und vorzugsweise Styrol in Betracht. Beispiele für Nitrile sind Acrylnitril und Methacrylnitril.

Die Vinylhalogenide sind mit Chlor, Fluor oder Brom substituierte ethylenisch ungesättigte Verbindungen, bevorzugt Vinylchlorid und Vinylidenchlorid.

Als Vinylether zu nennen sind z.B. Vinylmethylether oder Vinylisobutylether. Bevorzugt wird Vinylether von 1 bis 4 C-Atome enthaltenden Alkoholen.

Als Kohlenwasserstoffe mit 2 bis 8 C-Atomen und ein oder zwei olefinischen Doppelbindungen seien Ethylen, Propylen, Butadien, Isopren und Chloropren genannt.

Bevorzugte Hauptmonomere sind C₁-C₁₀-Alkyl(meth)acrylate und Mischungen der Alkyl(meth)acrylate mit Vinylaromaten, insbesondere Styrol (Polyacrylate).

Neben den Hauptmonomeren enthält das Polymer vorzugsweise weitere Monomere enthalten, z.B. Monomere mit einer Carbonsäure-, Hydroxyl- oder Aminogruppe. Der Gehalt dieser funktionellen Gruppen ermöglicht die Anbindung der erfindungsgemäßen Glycidester. Als derartige Monomere genannt seien z.B. Acrylsäure, Methacrylsäure, Maleinsäure, Itaconsäure, C1 bis C10 Hydroxylacrylate, Acrylamid oder Methacrylamid.

Zusammensetzungen, welche 2-Propylheptansäureglycidester, 4-Methyl-2-propylhexansäureglycidester oder deren Gemisch als Reaktivverdünner enthalten, haben insbesondere eine geringere Viskosität. Entsprechen haben auch Bindemittel, welche mit diesen Glycidestern modifiziert sind, eine geringere Viskosität.
Durch eine geringere Viskosität wird insbesondere auch ein höherer Feststoffgehalt bzw. eine höherer Gehalt an Pigmenten ermöglicht. Die Bindemittel, insbesondere auch Polyacrylatharze haben eine gute Untergrundbenetzung und Benetzung von Pigmenten.
Die Zusammensetzungen und Bindemittel haben gute anwendungstechnische Eigenschaften, wie Härte, Flexibilität und Glanz.

### Beispiele

### Vergleichsbeispiel 1

### Synthese von 2-Ethylhexansäureglycidester analog der Literaturvorschrift (J. Chem.Technol Biotechnol 74 ; 1145-1148, 1999)

Die Säure (72,0 g) wurde zusammen mit Chrom(III)acetat-hydroxid (0,10 g) im Kolben (250 ml 4-Halskolben mit Kühler, Tropftrichter, Magnetrührer) vorgelegt, erwärmt und nach Erreichen von 80°C das Epichlorhydrin (46,3 g) während einer Stunde zugetropft (leichte Wärmetönung). Nachdem über Nacht bei Temp. nachgerührt war, wurde das Gemisch unter Zugabe von Acetonitril (100 ml) mit trockenem K2CO3 (100 g) in einen 500 ml Kolben umgefüllt und am Rückfluss (= 82°C) 2 Arbeitstage gerührt. Das abgekühlte Gemisch wurde nach dem Filtrieren im Rotationsverdampfer eingeengt und der Rückstand 3 x mit Eiswasser ausgeschüttelt. Ausbeute: 65 %

### Vergleichsbeispiel 2

### Synthese von 2-Ethylhexansäureglycidester mit Cr(II)-ethylhexanoat als Katalysator

Die Säure (72,0 g) wurde zusammen mit Chrom(II)ethylhexanoat (0,24 g) im Kolben (250 ml 4-Halskolben mit Kühler, Tropftrichter, Magnetrührer) vorgelegt, erwärmt und nach Erreichen von 80°C das Epichlorhydrin (46,3 g) während einer Stunde zugetropft (leichte Wärmetönung). Nach 6 Std. Nachrührzeit wurde das Gemisch nach Zugabe von Acetonitril (100 ml) mit trockenem K2CO3 (69,1 g) in einen 500 ml Kolben umgefüllt und am Rückfluss (= 82°C) intensiv gerührt. Der Reaktionsverlauf wurde mit GC-Aufnahmen verfolgt. Da die Umsetzung von Chlorid zum Epoxid zum Stillstand kam wurden 20 g K2CO3 nachgegeben und 2 Arbeitstage weitergerührt. Der Reaktionsaustrag wurde nach dem Abtrennen der Salzreste (Glasfilternutsche) im Rotationsverdampfer eingeengt und der Rückstand im Vakuum (1,5 mbar) destilliert.

| | | |
|---|---|---|
| Fr.1 Kp = 90°C (1,5 mbar) | 95,5 Flä.-% | 2-Ethylhexansäure-glycidylester |
| | 1,0 Flä.-% | Isomeres |
| | 0,95 Flä.-% | Diolverb. aus Epoxid |
| | | |
| Fr.2 Kp = 90°C (1,5 mbar) | 96,0 Flä.-% | 2-Ethylhexansäure-glycidylester |
| | 1,0 Flä.-% | Isomeres |
| | 0,86 Flä.-% | Diolverb. aus Epoxid |
| Fr.3 Kp = 90°C (1,5 mbar) | 91,0 Flä.-% | 2-Ethylhexansäure-glycidylester |
| | 1,7 Flä.-% | Isomeres |
| | 3,0 Flä.-% | Diolverb. aus Epoxid |
| | | |
| Sumpf | 19,5 Flä.-% | 2-Ethylhexansäure-glycidylester |
| | 3,5 Flä.-% | Isomeres |
| | 24,0 Flä.-% | Diolverb. aus Epoxid |
| | 20 und 22 % | 2 unbekannte Verbindungen |

Fr.1 41,1 g
Fr.2 20,1 g
Fr.3 2,8 g
Sumpf 21,8 g

Summe: 95,8 g theor. Auswaage: 100,0 g
Ausbeute aus Fraktion 1 +2: 58,6 %

### Herstellungsbeispiel 1

### Synthese von 2-Propylheptansäureglycidester mit destillativer Aufarbeitung

Die Säure (86 g) wurde zusammen mit dem Chrom(III)acetat-hydroxid (0,10 g) im Kolben (500 ml 4-Halskolben mit Kühler, Intensivrührer, Tropftrichter) vorgelegt, erwärmt und nach Erreichen von 80°C das Epichlorhydrin (46,3) während einer Stunde zugetropft (leichte Wärmetönung). Nach 6 Std. Nachrührzeit wurde das Gemisch nach Zugabe von Acetonitril (100 ml) mit trockenem K2CO3 (100 g) am Rückfluss (= 82°C) gerührt. Der Reaktionsverlauf wurde mit GC-Aufnahmen verfolgt.
Ausbeute nach Destillation: 61 %

### Herstellungsbeispiel 2

### Synthese von 2-Propylheptansäureglycidester mit extraktiver Aufarbeitung

Die Säure (688 g) wurde zusammen mit dem Chromsalz (0,8 g) im Reaktor (3I - Doppelmantelreaktor mit Impellerrührer, sowie 2 Abgaskühlern hintereinander. Der Doppelmantel des Reaktor und die Abgaskühlung sind je mit Heiz-und/oder Kühlthermostaten ausgestattet. vorgelegt), erwärmt, nach Erreichen von 80°C das Epichlorhydrin (398 g) in 2 Stunden zugetropft und 24 Std. nachgerührt. Nach GC-Kontrolle wurde das vollständig umgesetzte Zwischenprodukt mit der Natronlauge (480 g, 50%ig) versetzt. Die Temperatur wurde dabei mit Hilfe der Zutropfgeschwindigkeit bei 55°C gehalten. Nach 2 Std. Nachrührzeit wurde mit 3 l Wasser verdünnt, 10 min bei Temp. gerührt und die beiden Phasen nach einer halben Stunde Absetzzeit getrennt. Die obere organische Phase wurde mit 2 mal mit Natriumsulfat getrocknet. Eine Wasserbestimmung nach dem Abfiltrieren des Trockenmittels zeigte 0,4 % Wasser, worauf bei 55°C Badtemperatur das Produkt im Stickstoffstrom weitergetrocknet wurde. Restliches Epichlorhydrin wurde durch Strippen mit N₂ für 3 Stunden bei 115°C entfernt.
Ausbeute: 66 %

### Anwendungsbeispiel

### Herstellung eines Acrylatharzes modifiziert mit 2-Propylheptansäureglycidester

In einen Laborreaktor mit indirekter Heizung (Wärmeträgeröl) oder regelbarer, elektrischer Widerstandsheizung; Produkttemperaturkontrolle; Schutzgaseinleitung, stufenlos regelbarem Rührer, zwei Zulaufbehältern und Rückflusskühler werden 298 g eines Lösemittels aus Alkylaromaten mit einem Siedebereich von 158 - 172°C und 241,6 g 2-Propylheptansäureglycidester eingewogen und unter Rühren aufgeheizt, dabei wird Schutzgas (Stickstoff) durchgeleitet.
Separat eingewogen werden 201,3 g Styrol, 145,0 g Methylmethacrylat, 147,4 g 2-Hydroxyethylmethacrylat und 70,1 g Acrylsäure, vorgemischt und in den ersten Zulaufbehälter gegeben (Monomermischung). Der zweite Zulaufbehälter erhält ein Gemisch aus 40,3 g des genannten aromatischen Lösemittels und 40,3 g tert.-Butylperethylhexanoat (Initiatorlösung). Der Inhalt des Laborreaktors wird auf 140°C aufgeheizt und kontinuierlich gerührt. Dann wir ein gleichmäßiger Zulauf der Initiatorlösung über eine Zeit von 4,75 Stunden gestartet. Eine Viertel Stunde nach dem Beginn des Initiatorzulaufs, wird mit dem gleichmäßigem Zulauf der Monomermischung begonnen. Über die gesamten Zulaufzeiten wird die Temperatur möglichst genau bei 140°C gehalten. Nach Ende des Monomerzulaufs wird der Initiatorzulauf noch eine halbe Stunde fortgesetzt. Nach Ende des Initiatorzulaufs wird mit Bestimmung des Festkörpers (EN ISO 3251, ca. 1,0 g, 15' 180°C) begonnen. Es wird bei 140°C gehalten bis der Festkörper über 71 % liegt und sich nicht mehr signifikant ändert, mindestens aber für drei Messungen. Dann wird abgekühlt und dann wird die entstandene Acrylatharzlösung ausgetragen. Mit 16,1 g des genannten aromatischen Lösemittel wird auf einen Festkörper (60' 130°C) von 70,0 ± 1,0 % eingestellt.
Das resultierende Acrylatharz hat eine Säurezahl von 6,8 mg KOH/g eine OH-Zahl von 148 mg KOH/g und eine Viskosität von 26,4 Pa·s (23°C)

Das erhaltene Acrylatharz ist besonders geeignet für die Herstellung von Zweikomponentenlacken, die als Härter Polyisocyanataddukte enthalten, zum Beispiel das Isocyanurat-Trimere des Hexamethylendiisocyanats. Sie zeigen aber auch gute Eigenschaften in anderen Lacksystemen.
Das hier beschriebene 2-Propylheptansäure-modifizierte Acrylatharz zeigt Vorteile gegenüber üblichen OH-Gruppen enthaltenden Acrylatharzen. Die Lacke daraus haben deutlich niedrigere Viskositäten gegenüber Acrylatharzen die unter gleichen Reaktionsbedingungen entstanden sind aber keine Glycidester-Modifikation in der beschriebenen Weise enthalten, und ermöglichen einen vergleichbar höheren Applikationsfestkörper, das bedeutet einen geringeren VOC-Wert. Die so modifizierten Acrylatharze erzeugen für die Lacksysteme bessere Pigmentbenetzung, bessere Verspritzbarkeit, bessere Untergrundbenetzung, besseren Glanz und Fülle der applizierten Filme. Die aus der Modifikation resultierenden unterschiedlich reaktiven OH-gruppen ergeben Vorteile für die Vernetzung in Bezug auf die Ausdehnung der molkularen Netzwerke im Lackfilm.
Im Vergleich zu den bereits bekannten Produkten auf Basis des Glycidesters der 2,2,3,5-Tetramethylhexansäure zeigen die Bindemittel auf Basis des Glycidesters der 2-Propylheptansäure niedrigere Lösungsviskositäten, die die Formulierung von Lacksystemen mit 5 - 10 % höherem Applikationsfestkörper ermöglichen (eine besondere, aktuelle Forderung der Lackindustrie zur Erfüllung der VOC-Regelungen), besseres Applikationsverhalten (Verlauf) und bessere Flexibilität. Die vergleichend erreichten Härtewerte der Lackerungen erfüllen die gestellten Forderungen.

## Patentansprüche

1. 2-Propylheptansäureglycidester, 4-Methyl-2-propylhexansäureglycidester oder deren Gemisch (zusammenfassend kurz als Glycidester bezeichnet).

2. Glycidester nach Anspruch 1, **dadurch gekennzeichnet, dass** es sich um ein Gemisch handelt, welches aus 1 bis 99 Gew.-% 2-Propylheptansäureglycidester und 1 bis 99 Gew.-% 4-Methyl-2-propylhexansäureglycidester, bezogen auf die Gewichtssumme von 2-Propylheptansäureglycidester und 4-Methyl-2-propylhexansäureglycidester, besteht.

3. Verfahren zur Herstellung der Glycidylester des Anspruchs 1 oder 2, **dadurch gekennzeichnet dass**
a) 2-Propylheptansäure, 4-Methyl-2-propylhexansäure oder deren Gemische mit Epichlorhydrin in Gegenwart eines Chromsalzes umgesetzt werden
b) die erhaltene Hydroxyverbindung in Gegenwart eines Alkali- oder Erdalkalihydroxids zum Glycidester umgesetzt wird und
c) das erhaltene Gemisch gegebenenfalls extraktiv aufgearbeitet wird.

4. Verwendung der Glycidester des Anspruchs 1 oder 2 als Reaktivverdünner.

5. Verwendung der Glycidester des Anspruchs 1 oder 2 als Reaktivverdünner für Zusammensetzungen, welche Epoxidverbindungen enthalten.

6. Härtbare, bei 21 °C, 1 bar flüssige Zusammensetzungen, enthaltend einen Glycidester nach Anspruch 1 oder 2.

7. Zusammensetzungen gemäß Anspruch 6, enthaltend mindestens 0,1 Gew.-% des Glycidester, bezogen auf alle Bestandteile der Zusammensetzung mit Ausnahme von Wasser und organischen Lösemitteln.

8. Verwendung der Glycidester des Anspruchs 1 oder 2 als Dispergier- oder Emulgierhilfsmittel.

9. Verwendung der Glycidester des Anspruchs 1 oder 2 als Dispergierhilfsmittel für Pigmente.

10. Pigmentzubereitungen, enthaltend 0,05 bis 20 Gew. Teile des Glycidesters nach Anspruch 1 oder 2, bezogen auf 100 Gew.-Teile Pigment.

11. Verwendung der Glycidester nach Anspruch 1 oder 2 zur Modifizierung von durch radikalische Polymerisation erhältlichen Polymeren, z.B. Polyacrylate, von Polykondensaten, z.B. Polyester, oder Polyaddukten, z.B. Polyurethane.

12. Durch radikalische Polymerisation erhältliche Polymeren, z.B. Polyacrylate, Polykondensaten, z.B. Polyester, oder Polyaddukten, z.B. Polyurethane, welche durch Umsetzung mit einem Glycidester nach Anspruch 1 oder 2 modifiziert sind.

## Claims

1. Glycidyl 2-propylheptanoate, glycidyl 4-methyl-2-propylhexanoate or a mixture of these (referred to collectively for short as glycidyl ester).

2. The glycidyl ester according to claim 1, comprising a mixture composed of 1% to 99% by weight of glycidyl 2-propylheptanoate and 1% to 99% by weight of glycidyl 4-methyl-2-propylhexanoate, based on the weight sum of glycidyl 2-propylheptanoate and glycidyl 4-methyl-2-propylhexanoate.

3. A process for preparing the glycidyl ester of claim 1 or 2, which comprises
a) reacting 2-propylheptanoic acid, 4-methyl-2-propylhexanoic acid or a mixture thereof with epichlorohydrin in the presence of a chromium salt,
b) reacting the resulting hydroxyl compound in the presence of an alkali metal hydroxide or alkaline earth metal hydroxide to give the glycidyl ester, and
c) if appropriate, working up the resulting mixture extractively.

4. The use of the glycidyl ester of claim 1 or 2 as a reactive diluent.

5. The use of the glycidyl ester of claim 1 or 2 as a reactive diluent for a composition comprising epoxide compounds.

6. A curable composition, liquid at 21°C and 1 bar, comprising a glycidyl ester according to claim 1 or 2.

7. The composition according to claim 6, comprising at least 0.1% by weight of the glycidyl ester, based on all constituents of the composition bar water and organic solvents.

8. The use of the glycidyl ester of claim 1 or 2 as a dispersing or emulsifying assistant.

9. The use of the glycidyl ester of claim 1 or 2 as a dispersing assistant for pigments.

10. A pigment preparation comprising 0.05 to 20 parts by weight of the glycidyl ester according to claim 1 or 2, based on 100 parts by weight of pigment.

11. The use of the glycidyl ester according to claim 1 or 2 for modifying polymers obtainable by free-radical addition polymerization, polyacrylates for example, polycondensates, polyesters for example, or polyadducts, polyurethanes for example.

12. A polymer obtainable by free-radical addition polymerization, polyacrylate for example, polycondensate, polyester for example, or polyadduct, polyurethane for example, which has been modified by reaction with a glycidyl ester according to claim 1 or 2.

## Revendications

1. Ester glycidique de l'acide 2-propylheptanoïque, ester glycidique de l'acide 4-méthyl-2-propylhexanoïque ou leur mélange (nommé en résumé ester glycidique).

2. Ester glycidique selon la revendication 1, **caractérisé en ce qu'**il s'agit d'un mélange qui est constitué de 1 à 99 % en poids d'ester glycidique de l'acide 2-propylheptanoïque et de 1 à 99 % en poids d'ester glycidique de l'acide 4-méthyl-2-propylhexanoïque, par rapport à la somme en poids de l'ester glycidique de l'acide 2-propylheptanoïque et de l'ester glycidique de l'acide 4-méthyl-2-propylhexanoïque.

3. Procédé de fabrication des esters glycidiques selon la revendication 1 ou 2, **caractérisé en ce que**
a) de l'acide 2-propylheptanoïque, de l'acide 4-méthyl-2-propylhexanoïque ou leurs mélanges sont mis en réaction avec de l'épichlorhydrine en présence d'un sel de chrome,
b) le composé hydroxy obtenu est mis en réaction en présence d'un hydroxyde alcalin ou alcalino-terreux pour former l'ester glycidique, et
c) le mélange obtenu est éventuellement traité par extraction.

4. Utilisation des esters glycidiques selon la revendication 1 ou 2 en tant que diluant réactif.

5. Utilisation des esters glycidiques selon la revendication 1 ou 2 en tant que diluant réactif pour des compositions qui contiennent des composés d'époxyde.

6. Compositions durcissables, liquides à 21 °C, 1 bar, contenant un ester glycidique selon la revendication 1 ou 2.

7. Compositions selon la revendication 6, contenant au moins 0,1 % en poids de l'ester glycidique, par rapport à tous les constituants de la composition à l'exception de l'eau et des solvants organiques.

8. Utilisation des esters glycidiques selon la revendication 1 ou 2 en tant qu'adjuvant de dispersion ou d'émulsification.

9. Utilisation des esters glycidiques selon la revendication 1 ou 2 en tant qu'adjuvant de dispersion pour pigments.

10. Préparations pigmentaires, contenant 0,05 à 20 parties en poids de l'ester glycidique selon la revendication 1 ou 2, par rapport à 100 parties en poids de pigment.

11. Utilisation des esters glycidiques selon la revendication 1 ou 2 pour la modification de polymères pouvant être obtenus par polymérisation radicalaire, p. ex. de polyacrylates, de polycondensats, p. ex. de polyesters, ou de polyadduits, p. ex. de polyuréthanes.

12. Polymères pouvant être obtenus par polymérisation radicalaire, p. ex. polyacrylates, polycondensats, p. ex. polyesters, ou polyadduits, p. ex. polyuréthanes, qui sont modifiés par réaction avec un ester glycidique selon la revendication 1 ou 2.
